# EUROPÄISCHE PATENTSCHRIFT

(11) **EP 0 926 240 B1**
(45) Veröffentlichungstag und Bekanntmachung des Hinweises auf die Patenterteilung: **05.03.2003**
(21) Anmeldenummer: 98122231.8
(22) Anmeldetag: 23.11.1998
(51) Int. Cl.: C12N 15/53, C12N 9/02, C12P 41/00

(54) **Mutanten der Formiatdehydrogenase aus Candida biodinii, Gensequenzen diese codierend sowie Verwendung dieser Formiatdeydrogenasen**
Candida biodinii Formate Dehydrogenase mutants, gene sequences encoding them and use of these Formate Dehydrogenase mutants
Mutants de la Formate Deshydrogénase de Candida biodinii, séquences géniques codant pour ces mutants, ainsi que leurs utilisations

(30) Priorität: 03.12.1997 DE 19753350
(43) Veröffentlichungstag der Anmeldung: 30.06.1999
(73) Patentinhaber: Degussa AG, 40474 Düsseldorf (DE)
(72) Erfinder: Kula, Maria-Regina, Prof. Dr., 52382 Niederzier (DE); Pohl, Martina, Dr., 52066 Aachen (DE); Slusarczyk, Heike, Dr., 52531 Übach-Palenberg (DE)

(56) Entgegenhaltungen:
- SAKAI Y ET AL: "Regulation of the formate dehydrogenase gene, FDH1, in the methylotrophic yeast Candida boidinii and growth characteristics of an FDH1-disrupted strain on methanol, methylamine, and choline." J BACTERIOL, JUL 1997, 179 (14) P4480-5, XP002123638 UNITED STATES
- TISHKOV ET AL.: 'Catalytic Properties and Stability of a Pseudomonas sp. 101 ...' BIOCHEM BIOPHYS RES COMMUN Bd. 192, 1993, Seiten 976 - 981

## Beschreibung

Die vorliegende Erfindung richtet sich auf Mutanten der Formiatdehydrogenase aus *Candida boidinii* (ATCC 32195). Weiterhin betrifft die Erfindung neue Gensequenzen, welche diese Mutanten codieren, sowie die Verwendung der erfindungsgemäßen Formiatdehydrogenasen in einem Verfahren zur Herstellung chiraler Verbindungen.

Zur Herstellung von L-Aminosäuren werden u. a. erfolgreich Biokatalysatoren eingesetzt. Ein Ansatz geht dabei von der Umwandlung prochiraler α-Ketosäuren durch reduktive Aminierung aus. Die dabei benutzten Aminosäuredehydrogenasen benötigen zur Umsetzung der α-Ketosäuren stöchiometrische Mengen an NADH bzw. NADPH als Coenzym. Diese Coenzyme sind sehr teuer und machen dadurch das o. g. Verfahren für den industriellen Maßstab wirtschaftlich uninteressant.

Eine Möglichkeit, hohe Kosten durch das Coenzym zu vermeiden, besteht darin, das Coenzym *in situ* zu regenerieren. Im technischen Maßstab wird derzeit u. a. die NAD-abhängige Formiatdehydrogenase aus der Hefe *Candida boidinii* im Emzymreaktor zur Coenzymregeneration eingesetzt.

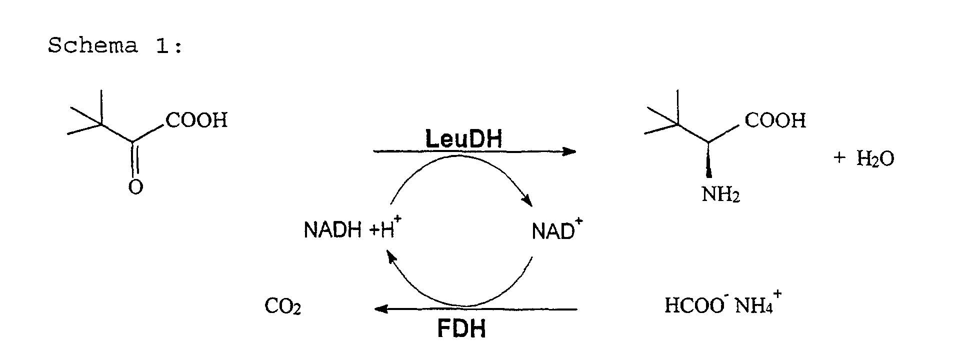

*In situ* Regeneration von NADH mit der NAD-abhängigen Formiatdehydrogenase bei der reduktiven Aminierung von Trimethylpyruvat zu L-tert-Leucin (Bommarius et al. Tetrahedron Asymmetry 1995, 6, 2851-2888).

Ein Nachteil, den der Einsatz der FDH aus *Candida boidinii* im Produktionsprozeß mit sich bringt, ist die Notwendigkeit, FDH während des Prozesses nachdosieren zu müssen, da sie aufgrund fehlender Stabilität inaktiviert. Diese Inaktivierung kann durch verschiedene Faktoren beeinflußt werden:
- pH-Wert
- Temperatur
- mechanische Belastung
- Ionenstärke und Ionenart der Substratlösung
- Spuren von Schwermetallen
- Oxidation von Sulfhydrylgruppen durch Luftsauerstoff
- Vernetzung durch Thiol-Disulfidaustausch

Tishkov et al. zeigten, daß mittels gerichteter Mutation der rekombinanten FDH aus *Pseudomonas sp.* 101 deren Stabilität gegenüber Quecksilbersalzen erhöht wird, wohingegen die thermische Stabilität jedoch durch die Mutagenese erniedrigt wurde (Biochem, Biophys. Res. Commun. 1993, 192, 976-981).

Sakai et al. klärten die Gensequenz der FDH aus der methylotrophen Hefe *Candida boidinii* auf (J. Bacteriol. 1997, 179, 4480-4485), die abgeleitete Proteinsequenz stimmt zu 100 % mit der Aminosäuresequenz der in dieser Arbeit zugrundegelegten rekombinanten FDH aus *Candida boidinii* überein (Sequenzprotokoll 1).

Angesichts des hierin angegebenen und diskutierten Standes der Technik war es mithin Aufgabe der vorliegenden Erfindung, die im technischen Prozeß eingesetzte FDH aus *Candida boidinii* derart abzuwandeln, daß diese verglichen mit der rekombinanten FDH und dem Wildtyp gegenüber der Oxidation eine größere Stabilität aufweist und so ein kostspieliges und kompliziertes Nachdosieren der FDH während des Prozesses überflüssig macht.

Diese und nicht näher genannte weitere Aufgaben werden durch die neuen Mutanten des Anspruchs 1 gelöst. Vorteilhafte Ausgestaltungen der Mutanten werden in Anspruch 2 unter Schutz gestellt. Auf die diese vorteilhaften Aminosäuresequenzen codierenden Gene richtet sich der Anspruch 3. Anspruch 4 enthält eine erfindungsgemäße Verwendung der mutierten FDHs.

Dadurch, daß man die rekombinante Formiatdehydrogenase aus *Candida boidinii* mittels gerichteter Mutagenese abwandelt, gelingt es sehr vorteilhaft und dennoch überraschend, gegenüber der rec-FDH und dem Wildtypenzym aggregationsund oxidationsunempfindlichere Mutanten zu generieren und so eine größere Standzeit dieser Enzyme im Produktionsprozeß herbeizuführen. Überraschenderweise werden dabei andere vorteilhafte Eigenschaften der FDH, wie z. B. katalytische Aktivität, Konformationsstabilität, Thermostabilität, etc., nur marginal beeinflußt, so daß der neue Vorteil durch andere hinzukommende Nachteile nicht negiert wird. Dies war keineswegs vorhersehbar, da in einem so komplexen Molekül häufig schon die kleinste Änderung zum vollständigen Verlust der Aktivität des Enzyms führt.

Bevorzugt wandelt man die betrachtete rekombinante Formiatdehydrogenase so ab, daß die schwefelhaltigen Aminosäuren des Enzyms unabhängig voneinander, einzeln oder zusammen gegen nicht schwefelhaltige Aminosäuren ausgetauscht werden.
Als ganz besonders bevorzugte Targets zur gerichteten Mutation erscheinen die Cysteine an den Positionen 23 und 262 der FDH. Dabei kann die gerichtete Mutagenese entweder nur an einer dieser Stellen oder an beiden geschehen. Vorteilhafterweise wird die schwefelhaltige Aminosäure der Position 23 und/oder 262 unabhängig voneinander, einzeln oder zusammen gegen Aminosäuren ohne Sulfhydrylrest ausgetauscht. Besonders bevorzugt ist ein Austausch gegen Serin, Alanin oder Valin.

Der Erfolg dieser Abwandlung war zum Zeitpunkt der Erfindungsfindung aus oben genanntem Grund weder vorhersehbar noch naheliegend.

Die durch die neuen Gensequenzen codierten Enzyme mit verbesserter Stabilität finden bevorzugt Anwendung in einem enzymatischen Verfahren zur Herstellung von chiralen Verbindungen u. a. der eingangs erwähnten Art.

Die erfindungsgemäßen Enzyme mit Formiatdehydrogenaseaktivität lassen sich vorteilhafterweise auf der Basis des rekombinanten FDH-Gens mittels gerichteter Mutagenese erzeugen und in *E. coli* exprimieren. Das Arbeiten mit rekombinanter FDH bietet den Vorteil, daß eine einheitliche Gensequenz und damit ein einheitliches Genprodukt vorliegt, in dem Mutationen erzeugt werden können. Um die Auswirkungen der Mutation in der Mutante wirkungsvoll mit dem Wildtypenzym vergleichen zu können, ist es aber von entscheidendem Vorteil, von einem einheitlichen Enzym ausgehen zu können. In *Candida boidinii* selbst liegen wahrscheinlich mehrere Isoformen des Enzyms vor, welche präparativ schlecht zu trennen sind. Auf jeden Fall zeigt das Wildtypenzym auf Proteinebene Mikroheterogenität.

Darüber hinaus kann man sich aller bezüglich *Escherichia coli* dem Fachmann bekannten Vorteile gegenüber den Ursprungsorganismen, wie z. B. hinsichtlich der Vermehrung oder der Expression, bedienen.

Die erfindungsgemäßen Gen- und Aminosäuresequenzen lassen sich nach an sich bekannten Methoden der Biochemie und Molekularbiologie herstellen.

So kann die genomische DNA aus *Candida boidinii* nach Kultivieren, Aufschließen und Fällen gemäß Ferbeyre et al. (Bio Techniques 1993, 14, 386) erhalten werden. Hieraus kann das FDH-Gen mittels Polymerasekettenreaktion (PCR) amplifiziert werden. Die notwendigen Primer wurden aus Proteinsequenzdaten abgeleitet. Das erhaltene FDH-Gen wurde in einen Klonierungsvektor ligiert und in *E. coli* transformiert. Nach Isolierung der rekombinanten Plasmid-DNA aus den *E. coli*-Zellen mittels eines kommerziell erhältlichen Präparationskits (z.B. Qiagen Plasmid Tip 20), wurden beide DNA-Stänge sequenziert. Die Sequenz ist in Sequenzprotokoll 1 dargestellt.

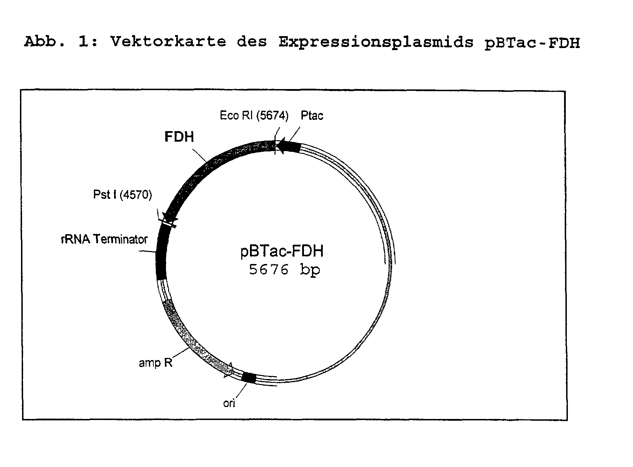

Die rekombinante Plasmid-DNA diente ebenfalls als Templat für die PCR-vermittelte Mutagenese nach Ho et al. (Gene, 1989,77,52-59). Die verwendeten Primer beinhalten das veränderte Codon (in Klammern) zum Austausch der entsprechenden Aminosäureposition: C23 (TGT Bp 67-69) gegen S23 (TCT); C262 (TGT, Bp 784-786) gegen V262 (GTT) oder A262 (GCT). Die amplifizierten, mutierten FDH-Gene wurden in den Expressionsvektor pBTac2 (Boehringer) (Abb. 1) kloniert und in *E. coli* exprimiert. Die Muteine wurden durch Aufschluß der kultivierten *E. coli* Zellen in Form eines zellfreien Rohextrakts gewonnen.

Der Vorteil der neuen Enzyme wird durch Stabilitätsuntersuchungen deutlich. In einer vergleichenden Untersuchung wurde die Inaktivierung der rekombinanten FDH aus *Candida boidinii,* der FDH-C23S, der FDH-C23S/C262A, der FDH-C23S/C262V, der FDH-C262V gemessen und deren Inaktivierungshalbwertszeiten gegenübergestellt.
Das Ergebnis ist in Tabelle 1 dargestellt.

**Tab. 1**

| Enzyme | Halbwertszeit (h) |
|---|---|
| recFDH | <21 |
| FDH-C23S/C262A | >750 |
| FDH-C23S | >750 |
| FDH-C23S/C262V | 160 |
| FDH-C262V | 21 |

Eindeutig ist die Verbesserung der Stabilisierung an der Erhöhung der Halbwertszeiten bei den Mutanten FDH-C23S/C262A sowie FDH-C23S und FDH-C23S/C262V zu erkennen. Die Schreibweise FDH-C23S bedeutet, daß bei der betrachteten Formiatdehydrogenase das Cystein (C) an Stelle 23 der Proteinsequenz durch Serin (S) ersetzt wurde. Analog ist der Terminus FDH-C262V, wo Cystein an Position 262 gegen Valin ausgetauscht wurde, zu verstehen.

Unter rec-FDH ist die rekombinante Formiatdehydrogenase zu verstehen, welche durch Klonierung und Expression des Gens aus *Candida boidinii* in *E. coli* gemäß unten stehender Vorschrift gewonnen werden kann.

Als Wildtypenzym wird die heterogene FDH, die aus *Candida boidinii* gewonnen werden kann, bezeichnet.

Die folgenden Beispiele sollen die Erfindung verdeutlichen.

### Beispiel 1: Herstellung genomischer DNA aus Candida boidinii

Die Präparation genomischer DNA aus der Hefe wurde in einer modifizierten Form nach Ferbeyre et al. (Bio Techniques 1993, 14, 386) durchgeführt. Dazu wurden die *Candida boidinii*-Zellen in 200 ml YEPD-Medium bei 30°C und 200 rpm bis zur späten logarithmischen Wachstumsphase kultiviert und anschließend durch Zentrifugation (10 min, 15°C, 5000 rpm, GSA-Rotor) geerntet. Unter diesen Bedingungen konnten ~ 2.0 g Zellfeuchtmasse / 100ml Kultur erzielt werden. Die Zellen wurden einmal mit 10 mM Citratphosphatpuffer, pH 7.5, gewaschen und anschließend in 10 ml Lysispuffer resuspendiert. Der Zellsuspension wurde 1 mg Protease [Qiagen] und 200 Units Lyticase aus *Arthrobacter luteus* [Sigma] pro ml Lysispuffer zugesetzt. Die Suspension wurde 60 min bei 37°C inkubiert und im Anschluß mit demselben Volumen (Vol.) Phenol-Chloroform-Isoamylalkohol (PCI) extrahiert. Nach 30-minütiger Zentrifugation bei RT und 12000 rpm im SS34 Rotor wurde die wässrige Phase abgenommen und bei Auftreten einer großen Interphase erneut mit PCI extrahiert. Die DNA wurde dann in der wässrigen Phase mit 1/10 Vol. 3 M Natriumacetat pH 5.2 und 2 Vol. eiskaltem Ethanol (abs.) gefällt, 5 min auf Eis gestellt, auf einen Glasstab gewickelt und unter der Sterilbank getrocknet. Nach dem Trocknen wurde die DNA über Nacht in 5 ml TE, pH 7.5, bei 4°C gelöst. Die RNA in der DNA-Präparation wurde durch Zugabe von 100 µg RNAseA / ml Lösung und Inkubation für 60 min bei 37°C unter leichtem Schütteln auf einem Horizontalschüttler (40 rpm) verdaut. Anschließend wurde die RNAseA durch Extraktion mit einem Vol. PCI gefällt und die wässrige Phase einmal mit einem Vol. CI extrahiert, um Phenolreste zu entfernen. Nach Zentrifugation wurde die DNA aus der wässrigen Phase mit 1/10 Vol 3M Natriumacetat pH 5.2 und 0.7 Vol. 2-Propanol bei RT gefällt, auf einen Glasstab gewickelt und wie zuvor getrocknet. Die genomischen DNA (gDNA) wurde über Nacht in 2.5 ml TE, pH 7.5 bei 4°C gelöst.
Die gDNA wurde anschließend in einem 0.5 %-igen Agarosegel hinsichtlich der Größenverteilung analysiert und durch Bestimmung der OD260nm und OD280nm quantifiziert und qualifiziert.
Mit dieser Methode konnte hochmolekulare und für die meisten molekularbiologischen Anwendungen ausreichend saubere genomische DNA in guter Ausbeute (700 µg genomische DNA / g Zellfeuchtmasse) gewonnen werden.

| Zusammensetzung der verwendeten Medien und Puffer: | | |
|---|---|---|
| YEPD-Medium: | 1 % (w/v) | Hefeextrakt |
| | 2 % (w/v) | Pepton |
| | 2 % (w/v) | Glucose |
| | | |
| Lysispuffer: | 10 mM | Citratphosphat pH 7.5 |
| | 1 M | Sorbitol |
| | 100 mM | EDTA |
| | 1 % (w/v) | SDS |
| | 1 % (v/v) | β-Mercaptoethanol |
| | | |
| TE pH 7.5: | 10 mM Tris-HCl, pH 7.5 | |
| | 1 mM EDTA | |
| | | |
| PCI | Phenol / Chloroform / Isoamylalkohol (25 : 24 : 1) | |
| | | |
| CI | Chloroform / Isoamylalkohol (24 : 1) | |

### Beispiel 2: Amplifizierung des FDH-Gens mittels PCR ausgehend von genomischer DNA

Alle PCR-Ansätze wurden mit 50 - 100 µl leichtem Mineralöl [Sigma] überschichtet und die PCR wurde mit Hilfe eines automatischen DNA-Thermal-Cyclers [Robocycler, Stratagene] nach folgendem Programm durchgeführt:

### PCR-Programm:

2 min Denaturierung bei 94°C (1x zu Anfang des Programms) 1 min Denaturierung bei 94°C
1.5 min Annealing der Primer bei 46 - 60°C (je nach Schmelztemperatur der Primer)
1.5 min Extension bei 72°C (Verlängerung der Primer durch die Taq-Polymerase)
zyklische Wiederholung der letzten drei Schritten (25 - 30 x)
10 min Extension bei 72°C, um zu gewährleisten, daß alle amplifizierten Fragmente vollständig verlängert werden

Der PCR-Ansatz enthielt:
100 ng gDNA
20 pmol Primer N-TermF3
20 pmol Primer C-TermR5
je 0.2 mM dNTPs
0.5 µl Taq-Polymerase (Boehringer)
10 µl Puffer 10x (Boehringer)
ad 100 µl mit A. dest
Annealingtemperatur: 48°C, 35 Zyklen

Das PCR-Fragment wurde mittels Sure-Clone®-Kits (Pharmacia, Freiburg) in den Vektor pUC18 ligiert. Zur Transformation wurde die Methode nach Hanahan (J. Mol. Biol. 1983, 166, 557) angewandt. Dazu wurden 2 µl Ligationsansatz Vektor pUC-FDH zu 100µl kompetenten *E. coli* XL1Blue-Zellen gegeben.

### Beispiel 3: Herstellung der Mutante FDH-C23S

Die Punktmutanten der FDH wurden auf der Basis des klonierten FDH-Gens (pUC-FDH) (s. Beisp. 1) nach der Methode von Ho et al. (Gene 1989, 77, 52-59) erzeugt. Dabei wurden folgende "innere" Oligonukleotid-Primer verwendet, die beide die Mutation beinhalten:
- innere Primer zur Einführung der Mutation C23S:

Als "äußere" Primer wurden folgende Oligonucleotid-Primer verwendet:

### 1. Herstellung der einzelnen Fragmente:

Ansatz A: Herstellung des SER23Sl-CTERMR5/Pstl- Fragments (1.0 kb)
   100ng pUC-FDH (1.1 kbFDH-EcoRI/Pstl in pUC18)
   30 pmol Primer SER23Sl
   30 pmol Primer CTERMR5/Pstl
   1.5 µl Pfu-Polymerase (2.5 U/µl)
   1/10 Vol Polymerasepuffer 10x
   je 0.2 mM dNTP
   ad 100 µl mit A. dest. Annealing-Temperatur: 46°C, 30 Zyklen
Ansatz B: Herstellung des PUC18S1-SER23AS1 - Fragments (500 bp)
   100 ng pUC-FDH (s.o.)
   30 pmol Primer PUC 18Sl
   30 pmol Primer SER23ASl
   1.5 1µl Pfu-Polymerase (s.o.)
   1/10 Vol Polymerasepuffer 10x
   je 0.2 mM dNTP
   ad 100 µl mit A. dest. Annealing-Temperatur: 44°C, 30 Zyklen.

Nach der PCR wurden die Ansätze in einem präparativem Agarosegel (1 %) aufgetrennt,
die Banden ausgeschnitten, mit Hilfe des Jetsorb®-Gelextraction-Kits (Genomed) isoliert, die Konzentrationen in einem analytischen Agarosegel abgeschätzt (Referenz: 1µg kb-Leiter, Gibco) und als Templat in der Fusions-PCR mit überlappenden Fragmenten eingesetzt.

### 2. Fusions-PCR zur Herstellung des kompletten FDH-C23S Gens (1.1 kb)

150 ng SER23S1-CTERMR5/Pstl-Fragment
90 ng PUC18Sl -SER23ASl -Fragment
20 pmol Primer NTERMF3/EcoRl
20 pmol Primer CTERMR5/Pstl
1.5 µl Pfu-Polymerase (2.5 U/µl)
1/10 Vol Polymerasepuffer 10x
je 0.2 mM dNTP
ad 100 µl mit A. dest.
Annealing-T.: 46°C 30 Zyklen

Der PCR-Ansatz wurde direkt im A-tailing (s.u.) eingesetzt.

### 3. Klonierung des PCR-Produkts in pMOS-Blue:

Die Klonierung erfolgte mittels des pMOS Blue T-Vektor-Kits (Amersham)

### a ) A-Tailing

100 µl Fusions-PCR-Ansatz wurden mit 1 Vol Chloroform-Isoamylalkohol (CI) (24:1) extrahiert.
25 µl wässrige Phase = 1/4 des PCR-Ansatzes
1.8 µl 10 x Puffer (siehe Protokoll des Amersham)
1.8 µl dNTP-Mix (siehe Protokoll des Amersham)
8.5 µl A-tailing-Puffer (siehe Protokoll des Amersham)
0.5 µl Tth-DNA-Polymerase
ad 85 µl mit A. dest
15 min 70°C
1 x mit CI extrahieren

Nach Isolierung des das FDH-C23S-Gen umfassenden PCR-Fragments mittels Agarosegelelektrophorese und Isolation des PCR-Fragments mittels Jet-Sorb (Genomed), wurde das Fragment in den Vektor pMOS-Blue ligiert.

### Ligation in pMOS-Blue:

50 ng pMOS-Blue-Vektor
120 ng FDH-Ser23 - 3'dA
0.5 µl ATP 10 mM
1.0 µl Ligase-Puffer
0.5 µl DTT 100mM
0.5 µl T4 DNA-Ligase (2-3 Weiss-Units)
ad 10 µl mit A.dest

Inkubation über Nacht bei 16°C.

### b) Transformation in MOS Blue kompetente Zellen (Nach Amersham Protokoll, 1994)

1 µl Ligationsansatz
20 µl kompetente Zellen
40 sec 42°C
2 min auf Eis
80 µl LB-Medium zufügen 1 h 37°C
50 µl auf LB mit Ampicillin und Tetracyclin ausplattieren.

### 4. Klonierung des FDH-C23S-Gens in den Expressionsvektor pBTac2 (Boehringer)

Aus den rekombinanten pMOS-Blue-Zellen wurde nach Vermehrung in *E. coli* die Plasmid-DNA (pMOS-FDH-C23S) isoliert und das FDH-C23S-EcoR1/Pst1-Fragment (1.1 kb) mittels Restriktionsverdau präpariert.

### Präparativer EcoR1-Pst1-Verdau

15 µl Plasmid-DNA (ca. 200 ng) pMOS-FDH-C23S
3µl Puffer H (Boehringer)
0.5 µl EcoR1 (10U/µl)
0.5 µl Pst1 (10U/µl)
11 µl A. dest.
2 h, 37°C

Das Fragment wurde nach den o.a. Methoden isoliert.

### Ligation in pBTac2

100 ng pBTac2 wurde, wie zuvor beschrieben, mit EcoRI und Pst1 verdaut, und der linearisierte Vektor wurde nach den o.a. Methoden isoliert. In den geöffneten Vektor wurde das FDH-C23S-EcoR1/Pst1-Fragment (1.1 kb) ligiert.

Der Ligationsansatz enthielt:
45 ng pBTac2-EcoRI/Pst1
60 ng FDH-C23S-EcoRI/Pst1
1 µl Ligase Puffer 10x (Boehringer)
0.5 µl T4 DNA-Ligase (Boehringer)
ad 10 µl mit A. dest
Über Nacht bei 16°C inkubieren.

### 5. Transformation in E. coli JM 105

Zur Transformation wurden 5 µl Ligationsansatz mit 100 µl kompetenten *E. coli* JM 105-Zellen versetzt und die Transformation nach Hanahan (s.o.) durchgeführt.

### Beispiel 4: Expression von FDH-C23S in E. coli

Das rekombinante Wildtyp-FDH-Gen und die FDH-Mutanten wurden im 200 ml-Maßstab in *E. coli* Zellen JM105 exprimiert. 200 ml LB-Medium wurden zur Selektion 100 µg Ampicillin pro ml zugesetzt und im Verhältnis 1:50 mit einer über Nacht inkubierten Vorkultur angeimpft. Die Zellen wurden bei 37°C und 180 rpm auf einem Reziprokschüttler kultiviert und bei einer optischen Dichte (OD550nm) von 0.6 - 0.8 mit 1mM IPTG induziert. Die Expressionsdauer betrug zwischen 5.0 h (0.7 g Zellfeuchtmasse) und 20 h (1.25 g Zellfeuchtmasse). Die Zellen wurden durch Zentrifugation (10 min, GSA-Rotor, 10 000 rpm) geerntet.
- LB (Luria Bertani)-Medium:: 1.0 % Bacto - Trypton 0.5 % Bacto - Hefeextrakt 1.0 % NaCl
mit NaOH auf pH 7.5 einstellen
- LBamp-Medium: LB-Medium mit 100 µg/ml Ampicillin

Der Zellaufschluß erfolgte mechanisch mittels Glasperlen (Durchmesser 0.3 mm). Es wurden 30%-ige *E. coli*-Zellsuspension in Aufschlußpuffer hergestellt, denen die doppelten Gewichtsanteile an Glasperlen zugefügt wurden. Der Aufschluß erfolgte je nach Volumen im 1 - 2 ml-Maßstab in einer Schwingmühle [Retsch; Hummel und Kula (1989)
J. Microbiol. Meth. 9, 210], einem SS34-Zentrifugenröhrchen (10-20 ml) auf einem Vortex [IKA] oder in einem Desintegrator S (20 - 50 ml) [IMA] über einem Zeitraum von 20 min . Der Aufschluß wurde 10 min bei 10000 rpm und 4°C zentrifugiert, der zellfreie Überstand abgenommen und die Glasperlen und das Zellpellet einmal mit ¼ - ½ Zellsuspensionsvolumen Puffer gewaschen. Nach erneuter Zentrifugation wurden die zellfreien Überstände vereinigt. Die Volumenaktivität der FDH-C23S im zellfreien Rohextrakt betrug 10 U/ml.
- Aufschlußpuffer:: 100 mM Kaliumphosphatpuffer, pH 7.5
10 Tropfen Ucolup / L Puffer

### Beispiel 5: Bestimmung der Stabilität der FDH-C23S

Zur Bestimmung der Halbwertszeiten der Deaktivierung wurde der zellfreie Rohextrakt der FDH-C23S verwendet.

Die Testansätze enthielten: 0.05-0.5 M NH₄-Trimethylpyruvat
0.1-1 M L-tert. Leucin
2.7 M NH₄-Formiat
0.5 U/ml FDH-C23S
pH 6-9
T = 40°C

Die Inkubation erfolgte über 18 Tage. Täglich wurden Proben für den Aktivitätstest entnommen. Die halblogarithmische Auftragung der Restaktivität gegen die Zeit ergaben eine Gerade, deren Steigung die Inaktivierungskonst. k darstellt. Die Halbwertszeit τ der Inaktivierung erhält man aus dem Zusammenhang τ=ln2/k.

## Patentansprüche

1. Gegenüber der rekombinanten Formiatdehydrogenase und dem Wildtypenzym aggregations- und oxidationsunempfindlichere Mutanten der Formiatdehydrogenase gemäß SEQ ID NO:1
**dadurch gekennzeichnet,**
**daß** man an der Position 23 die Aminosäure Cystein der rec-FDH durch gerichtete Mutagenese gegen Serin, Alanin oder Valin austauscht.

2. Mutanten nach Anspruch 1,
**dadurch gekennzeichnet,**
**daß** man an der Positionen 262 die Aminosäure Cystein gegen Serin, Alanin oder Valin eintauscht.

3. Gene, die Mutanten nach Anspruch 1 und/oder 2 codierend.

4. Verwendung der Mutanten nach Anspruch 1 in einem Verfahren zur Herstellung chiraler Verbindungen.

## Claims

1. Mutants of formate dehydrogenase in accordance with SEQ ID NO: 1 which are more stable to aggregation and oxidation than recombinant formate dehydrogenase and the wild type enzyme, **characterised in that** the amino acid cysteine at position 23 in rec-FDH is replaced by serine, alanine or valine by means of targeted mutagenesis.

2. Mutants according to Claim 1, **characterised in that** the amino acid cysteine at positions 262 is exchanged for serine, alanine or valine.

3. Genes which code for the new mutants according to Claim 1 and/or 2.

4. Use of the mutants according to Claim 1 in a process for preparing chiral compounds.

## Revendications

1. Mutants de la formiate déshydrogénase conformément à SEQ ID n° 1 plus insensible par rapport aux formiate déshydrogénases recombinants et à l'enzyme de type sauvage, agrégation et à l'oxydation,
**caractérisés en ce qu'**
on échange à la position 23 l'aminoacide cystéine de la FDH recombinante par mutagénèse dirigée, contre de la sérine, de l'alamine ou de la valine.

2. Mutants selon la revendication 1,
**caractérisés en ce qu'**
on échange à la position 262 l'aminoacide de cystéine contre de la serine, de l'alanine ou de la valine.

3. Gènes qui codent pour les mutants selon la revendication 1 et/ou 2.

4. Utilisation des mutants selon la revendication 1 dans un procédé d'obtention de composés chiraux.


### SEQUENZPROTOKOLL

(1) ALLGEMEINE ANGABEN:
   (i) ANMELDER:
      (A) NAME: Degussa Aktiengesellschaft
      (B) STRASSE: Weissfrauenstrasse 9
      (C) ORT: Frankfurt am Main
      (E) LAND: Germany
      (F) POSTLEITZAHL: D-60311
      (G) TELEFON: 069-218-01
   (ii) BEZEICHNUNG DER ERFINDUNG: Neue Mutanten der Formiatdehydrogenase aus Candida boidinii, neu Gensequenzen diese codierend sowie Verwendung der neuen Fromiatdehydrogenasen
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 1095 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: rec-FDH
      (C) INDIVIDUUM/ISOLAT: Candida boidinii
      (G) ZELLTYP: Hefe
   (viii) POSITION IM GENOM:
      (C) EINHEITEN: bp
   (ix) MERKMAL:
      (A) NAME/SCHLÜSSEL: CDS
      (B) LAGE:1..1095
   (xi) SEQUENZBESCHREIBUNG: SEQ ID NO: 1:
   ATG AAG ATT GTC TTA GTT CTT TAT GAT GCT GGT AAG CAC GCT GCT GAT       48
   Met Lys Ile Val Leu Val Leu Tyr Asp Ala Gly Lys His Ala Ala Asp
    1               5                  10                  15
   GAA GAA AAA TTA TAT GGT TGT ACT GAA AAT AAA TTA GGT ATT GCC AAT       96
   Glu Glu Lys Leu Tyr Gly Cys Thr Glu Asn Lys Leu Gly Ile Ala Asn
               20                  25                  30
   TGG TTA AAA GAT CAA GGT CAT GAA CTA ATT ACT ACT TCT GAT AAA GAA      144
   Trp Leu Lys Asp Gln Gly His Glu Leu Ile Thr Thr Ser Asp Lys Glu
           35                  40                  45
   GGT GAA ACA AGC GAA TTG GAT AAA CAT ATC CCA GAT GCT GAT ATT ATC      192
   Gly Glu Thr Ser Glu Leu Asp Lys His Ile Pro Asp Ala Asp Ile Ile
       50                  55                  60
   ATC ACC ACT CCT TTC CAT CCT GCT TAT ATC ACT AAG GAA AGA CTT GAC      240
   Ile Thr Thr Pro Phe His Pro Ala Tyr Ile Thr Lys Glu Arg Leu Asp
   65                  70                  75                  80
   AAG GCT AAG AAC TTA AAA TTA GTC GTT GTC GCT GGT GTT GGT TCT GAT      288
   Lys Ala Lys Asn Leu Lys Leu Val Val Val Ala Gly Val Gly Ser Asp
                   85                  90                  95
   CAC ATT GAT TTA GAT TAT ATT AAT CAA ACA GGT AAG AAA ATC TCA GTC      336
   His Ile Asp Leu Asp Tyr Ile Asn Gln Thr Gly Lys Lys Ile Ser Val
               100                 105                 110
   CTG GAA GTT ACA GGT TCT AAT GTT GTC TCT GTT GCT GAA CAC GTT GTC      384
   Leu Glu Val Thr Gly Ser Asn Val Val Ser Val Ala Glu His Val Val
           115                 120                 125
   ATG ACC ATG CTT GTC TTG GTT AGA AAT TTC GTT CCA GCA CAT GAA CAA      432
   Met Thr Met Leu Val Leu Val Arg Asn Phe Val Pro Ala His Glu Gln
       130                 135                 140
   ATT ATT AAC CAC GAT TGG GAG GTT GCT GCT ATC GCT AAG GAT GCT TAC      480
   Ile Ile Asn His Asp Trp Glu Val Ala Ala Ile Ala Lys Asp Ala Tyr
   145                 150                 155                 160
   GAT ATC GAA GGT AAA ACT ATC GCT ACC ATT GGT GCT GGT AGA ATT GGT      528
   Asp Ile Glu Gly Lys Thr Ile Ala Thr Ile Gly Ala Gly Arg Ile Gly
                   165                 170                 175
   TAC AGA GTC TTG GAA AGA TTA CTC CCA TTT AAT CCA AAA GAA TTA TTA      576
   Tyr Arg Val Leu Glu Arg Leu Leu Pro Phe Asn Pro Lys Glu Leu Leu
               180                 185                 190
   TAC TAC GAT TAT CAA GCT TTA CCA AAA GAA GCT GAA GAA AAA GTT GGT      624
   Tyr Tyr Asp Tyr Gln Ala Leu Pro Lys Glu Ala Glu Glu Lys Val Gly
           195                 200                 205
   GCT AGA AGA GTT GAA AAT ATT GAA GAA TTA GTT GCT CAA GCT GAT ATC       672
   Ala Arg Arg Val Glu Asn Ile Glu Glu Leu Val Ala Gln Ala Asp Ile
       210                 215                 220
   GTT ACA GTT AAT GCT CCA TTA CAC GCA GGT ACA AAA GGT TTA ATT AAT       720
   Val Thr Val Asn Ala Pro Leu His Ala Gly Thr Lys Gly Leu Ile Asn
   225                 230                 235                 240
   AAG GAA TTA TTA TCT AAA TTT AAA AAA GGT GCT TGG TTA GTC AAT ACC       768
   Lys Glu Leu Leu Ser Lys Phe Lys Lys Gly Ala Trp Leu Val Asn Thr
                   245                 250                 255
   GCA AGA GGT GCT ATT TGT GTT GCT GAA GAT GTT GCA GCA GCT TTA GAA       816
   Ala Arg Gly Ala Ile Cys Val Ala Glu Asp Val Ala Ala Ala Leu Glu
               260                 265                 270
   TCT GGT CAA TTA AGA GGT TAC GGT GGT GAT GTT TGG TTC CCA CAA CCA       864
   Ser Gly Gln Leu Arg Gly Tyr Gly Gly Asp Val Trp Phe Pro Gln Pro
           275                 280                 285
   GCT CCA AAG GAT CAC CCA TGG AGA GAT ATG AGA AAT AAA TAT GGT GCT       912
   Ala Pro Lys Asp His Pro Trp Arg Asp Met Arg Asn Lys Tyr Gly Ala
       290                 295                 300
   GGT AAT GCC ATG ACT CCT CAC TAC TCT GGT ACT ACT TTA GAC GCT CAA       960
   Gly Asn Ala Met Thr Pro His Tyr Ser Gly Thr Thr Leu Asp Ala Gln
   305                 310                 315                 320
   ACA AGA TAC GCT GAA GGT ACT AAA AAT ATT TTG GAA TCA TTC TTT ACC      1008
   Thr Arg Tyr Ala Glu Gly Thr Lys Asn Ile Leu Glu Ser Phe Phe Thr
                   325                 330                 335
   GGT AAA TTT GAT TAC AGA CCA CAA GAT ATT ATC TTA TTA AAT GGG GAA      1056
   Gly Lys Phe Asp Tyr Arg Pro Gln Asp Ile Ile Leu Leu Asn Gly Glu
               340                 345                 350
   TAC GTT ACT AAA GCT TAC GGT AAA CAC GAT AAG AAA TAG                  1095
   Tyr Val Thr Lys Ala Tyr Gly Lys His Asp Lys Lys  *
           355                 360                 365